# EUROPEAN PATENT APPLICATION

(11) **EP 1 114 629 A2**
(43) Date of publication of application: **11.07.2001**
(21) Application number: 00500179.7
(22) Date of filing: 04.08.2000
(51) Int. Cl.: A61F 9/06

(54) **Mechanical visual protector for welding application**

(30) Priority: 06.08.1999 CL 99049821
(71) Applicant: Sanchez Talero, John Alejandro, Santafe de Bogota D.C., Cundinamarca (CO)
(72) Inventor: Sanchez Talero, John Alejandro, Santafe de Bogota D.C., Cundinamarca (CO)
(74) Representative: Cobas Horcajo, Susana

(57) **Abstract**

Visual Protector to be used in welding application which filter-holder window opens Wire Rope-Lever, Electro-Magnet-Lever, Pneumatic-Lever, or Hydraulic-Lever, driven by several different devices, located at the same electrode-holder pliers on the floor. Filter-holder window is shut off by tension springs located between the filter-holder and its base, allowing to open and shut at will and quickly the filter-holder window preventing visuals from direct exposure to welding light, this way lowering visual accidents, application time, and error margins, increasing welding application efectiveness and quality.

## Description

When applying welding, a face shield is used, which filter-holder window is manually operated, involving the whole arm, and some times more importance is given to lower the face shield than to welding application itself, thus resulting in visual direct and undeliberately exposure to welding light.

Usually, when pointing to the target, since it is troublesome to lower and lift up visual protector in each welding point, most welders choose to refrain from using the face shield, considering that it will suffice shutting the visuals and turn the face to keep from be injured by the rays coming from weld light.

It is also laborious to handle the face shield when hands are used, e.g., when applying welding with addition of inox. Steel (Argon).

Usually, when welding is applied, the point to begin welding is located and visual protector is operated with the arm, and the movement is not properly targeted, beginning application in a site other than required.

As for visual protectors with lens which get dark with welding light contact, in spite of the fast darkening of lens, never it will be higher or equal to light velocity.

### THE OBJECTIVE OF INVENTION

The purpose of this invention shall be obviate the inconvenience above referred to by using the visual protector outfitted with a mechanism to open and shut holder-filter window by operating at will several different means, with the hands or the foot.

This device allows for greatly lowering visual accidents due to eyes direct and undeliberately exposure to welding light, because owing to quick and undeliberately motions operator can shut before welding light and open for application, affording with more visual protection and thus drive the mechanisms as many times as required to apply welding in a more safe and fast manner, thus increasing welding application effectiveness and quality.

Additionally, the invention relies on means inhibiting energy flow to electrode-holder pliers of welding equipment while filter-holder window is open, restarting energy flow when such window is shut, thus preventing from undeliberately accidents to visual with welding light.

To more elucidate the invention advantages compared to the current known techniques, following the possible performance of its principles are described, supported by drawings and exhibits.

For such purposes, it is intended to provide with a visual protector according to claim 1.

### EXPLANATION OF FIGURES

Figure # 1 displays a perspective and schematic view of the visual protector for welding, which opens holder-filter window dark glass-holder through a mechanical device wire rope-lever located or installed in the electrode-holder pliers of welding equipment.

Figure # 2, shows a perspective and schematic view of the visual protector with its mechanical shutter device, filter-holder window shut by springs located between filter-holder window and its base; also shows in each of both two lids of the mechanical devise two tapered bars with drills to anchor and adjust to its base.

Figure # 3 shows a perspective and schematic view of its L-shaped base which adjusts and fastens shutting devices of mechanical, pneumatic or hydraulic visual protectors, with electrode-holder pliers of welding equipment or handles of cutting equipment which have not fixing screws.

Figure # 4 shows a perspective and schematic view clamp-shaped, which grasps and adjusts shutting devices of mechanical, pneumatic or hydraulic visual protectors, with electrode-holder pliers of welding equipment or handles of cutting equipment which have not holding screws.

Figure # 5 shows a perspective and schematic view of an open visual protector which opens its filter-holder window through a mechanical motion by using a pedal.

Figure # 6 shows a perspective and schematic side view of an visual protector which lifts up its filter-holder window by means of an air-or-oil driven dual-acting plug.

Figure # 7 displays a side and schematic view of the device designed to be manually operated acting on the several different shutting units of filter-holder windows of the pneumatic and hydraulic visual protectors; such units are: Bellow, diaphragm and plug.

Figure # 8 shows a perspective and schematic view designed to be foot operated by driving the different shutting units of holder-filter windows of pneumatic and hydraulic visual protectors; such units are: bellow, diaphragm and plug.

Figure # 9 shows a perspective and schematic view of an visual protector along with filter-holder window in open position, with a system to open the filter-holder window by means of the plug an electromagnet driven by electric or sun energy, since it is provided with solar panels located at the top or the bottom of filter-holder window. Pushing button is located in the pliers of welding or cutting handle, wireless for solar protector.

Figure # 10 shows a side view of a device coupled to electrode-holder pliers with its appropriate driving hand.

### DESCRIPTION OF INVENTION REFERRED TO FIGURES

Figure # 1 shows an visual protector (1) opened. This protector is interconnected to its mechanical device (2) through the sheath (3) and wire rope (4) which when contracted by the lever (5) pivoting in the point (6), when introduced in the plug (7) with the finger (8) the cylindrical-shaped flange(), is shrunk for the press-wire rope (10) to be fitted with no friction. This flange (9) is supported on pivoting point (11) which when shrinking by the device (2) opens of lifts up filter-holder window or dark holder-glass (12). In addition, this flange has a vertical slot (13) which allows to open holder-filter window (12) without changing the wire rope (4) directional shaft which opens between points 14 and 15 where there are some tension springs (16) which shut filter-holder window (12) when device (2) is not activated, but additionally filter-holder window (12) can be left opened with a slight motion by pressing forwards the plug (7) with the finger (8) inserting or joining the whetstone (17) in the box (18) showed by the line drawn (42), and to unhook or untie, press again with finger (8) the plug (7) pulling it out. With another slight motion, this time backwards, the filter-holder (12) is shut off again due to springs (16) when finger (8) is withdrawn. Additionally, in the device (2) a system may be added to energize or denergize electrode-holder pliers or cutting handle by means of a wireless switch (20) or battery giving the signal to a contactor in the welding or cutting equipment as soon as filter-holder is shut off to reassume energy flow by means of sensors (21 and 22) when they are confronted.

Figure # 2 shows an visual protector (1) closing filter-holder window (12) by tension-spring system (16) located between points 14 and 15 operating either with top or lateral (16) springs accurately gauged to allow for the filter-holder (12) to be opened and shut off if device (2) is not manually activated and the driver plug (7) returns to its initial position ready and in a position to be activated again by the finger (8). This device (2) is provided with a V-shaped lever (7) the ends with circular holes the same as its central point (23) where fixed shaft (6) pivots in the hole (24) it has a vertical slot (25) the same as that in the flange (9) allowing the wire rope (4) to be displaced without losing its steering shaft while filter-holder window (12) is opened or shut; it has in addition a cylindrical socket for to fits with slight friction. The other wire rope press (26) also spherical or cylindrical shaped with the appropriate grub screw (27), as that in wire rope head (10). This spherical shape of wire rope press (10 and 26) allow, during welding or cutting operation, to spin over its axis the electrode-holder pliers or handle of the entire cutting equipment together with the device (2) while keeping static or on the head the visual protector (1) neither throttling or failing the wire rope (4), nor becoming hard when driving the device (2) with finger (8) to open filter-holder window (12). The ends of the sheath (3) of the wire rope (4) interconnecting visual protector (1) to its driver device (2) rely on a coupling conventional system (28) allowing to keep them firmly joined and easy to rotate on its axis without any detaching, throttling or breakage.

According to Figure # 3 Device (2) includes a in the side lids (35) two tapered bars (33 and 34) to be anchored and with cavities (36) for adjustment either in the L-shaped or clamp shaped base. The function of the two tapered bars will be to locate the device to the other side of the electrode-holder pliers or cutting handle to locate the device depending on a right-handed or left-handed operator.

Figure # 4 shows the base (29) L-shaped and has the wing (30) with the parallel slots (31) to fasten electrode-holder with the screw (44). In the wing (32) the device has two tapered bars (37-38) of the base, to hold in place the device which fit in the tapered bars of the base holding in place in the way so-called dovetail in two positions. This anchor system (39) has three rectangular bars (40) each provided with a rectangular pin (41) lined up inserted in the sockets (36) thus affording with the needed adjustment. Such bars (40) are found attached to the base by one of the ends (42) and in the other end the three bars (40) are joined by a pulsing button (43) which when pushed hides pins (41) and unanchor, unpin or disengage such pins from the sockets or slots. And this ways several welders can use the same equipment with the appropriate protectors and adjust them at their discretion and comfortableness.

Explanation of invention according to Figure # 5 which shows rectangular base (45) which by means of the same system as in Fig. # 4 related to fastening system (31) joins the base (29) to the device (2) by means of the tapered bars (37-38) and bars (41) and pins (42), varies because has a single wing or base (45) and has is circular-shaped in the other side (46) where couples a circular conventional worm (47) joined to base (45) and circle-shaped in the other side (78) where couples one conventional circular worm (47) fastened to the base (45) by four screws (48) located in the ends, which then, by the clamps, (47) to tightly join, assemble or couple base (45) with the device (2) and the former to the electrode-holder or cutting handle (47) in Figure # 10. This base system (45) with clamps has been designated to mount or anchor the devices (2) on electrode-holder pliers or handles of cutting equipment which is not provided with setscrew.

Explanation of invention according to Figure # 6. This figure shows a visual protector (1) with filter-holder window (12) in open position, the same as in Figure # 1, although different because this is foot-driven by means of a device (50) operating the lever (51) on its (52) base that by means of point (53) opens the opposed side foot presses (54) shrinking the wire rope (4) inside the sheath (3) and is fastened to the base (52) by the spherical press-wire rope (10).

In reference to Figure # 7 you observe a visual protector (1) to apply weld with filter-holder window (12) opened by a dual-acting plug (55) located between the points (14 and 15). The plug (55) is expanded when loading with oil or air pressure through devices such as those in Figure # 8 when pressed, thus lifting up the filter-holder window (12) and automatically shuts through springs (16) located between points (14 and 15) when pressure on devices is stopped.

Figure # 8 shows a device (2) which manually triggers a visual protector (1) of Figure # 7 which filter-holder window is triggered or opened by air or oil pressure. This device (2) is outfitted with a lever (5) supported and pivoted on point 6 which when introduced the plug (7) shut the space between the points 56 and 57, thus contracting the several different protecting units of pressure, such as: bellow (58) for air, diaphragm (59) for air or oil, and plug (60) for air or oil; it has, in addition, a spring (61) which draw back the items above. They also have a top spherical base (62) where cylindrical pitchfork (63) of the end of the lever (5) is supported, as well as device (2) of Figure # 1 which has a grinder (17) inserted in the box (18) to leave open filter-holder window without requiring manual pressure. The side or lids of the device (2) in Fig # 3 with tapered bars (37-38) inserted in the base (29) of Figure # 4 or (45) in Figure # 5 allowing the adjustment required by the operator; it also has the same microswitch 20 system which sends the signal to a contactor fo welding or cutting equipment, thus reassuming energy flow, when sensors (21 and 22) are opposed each other.

Figure # 9 shows a pedal device (50) to be operated with foot (54) which triggers pneumatic or hydraulic visual protector as that in figure # 7. This device has a design allowing to couple pressure units as those in Fig. # 8. Such devices produce air or oil pressure when contrancted the space between the point 64 and 65 when pressing peda (51) making pivot lever (66) in pivot point (21). The way for the filter-holder window to be shut off is the same as in Fig. # 2, through springs (16) immediately pressure is released.

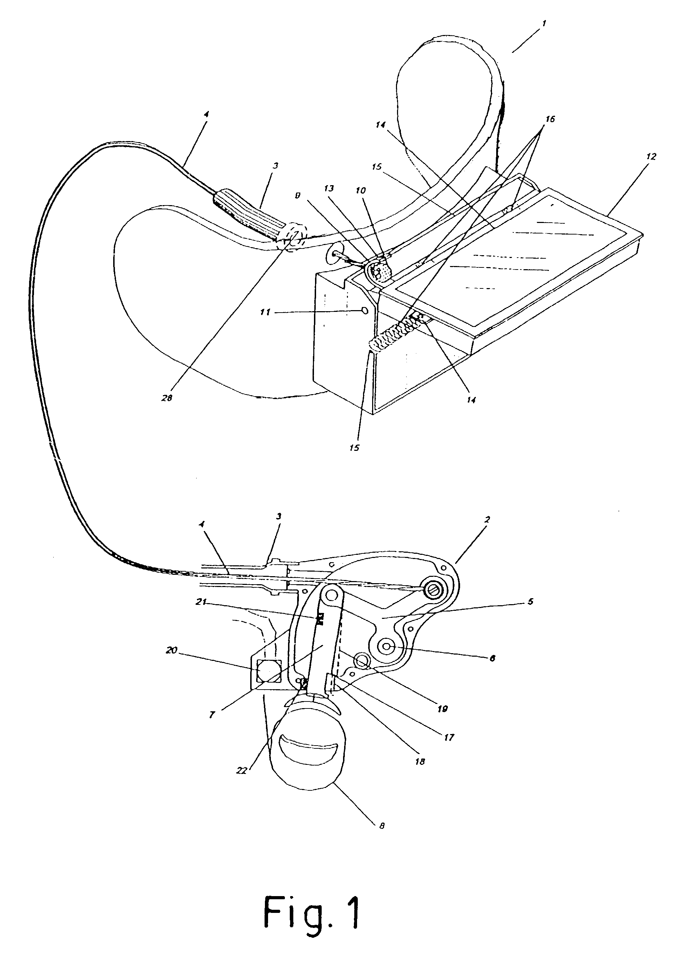

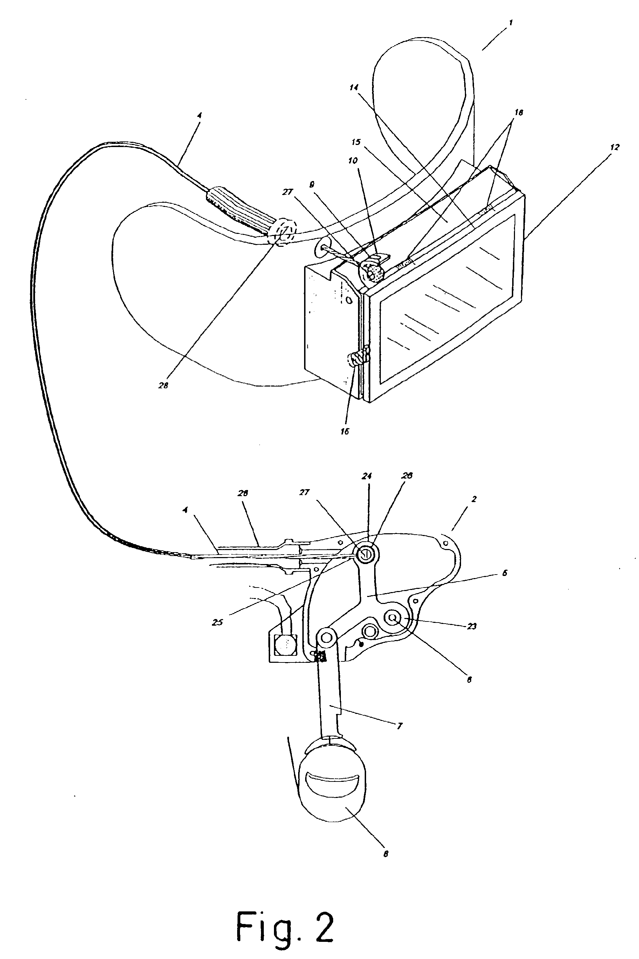

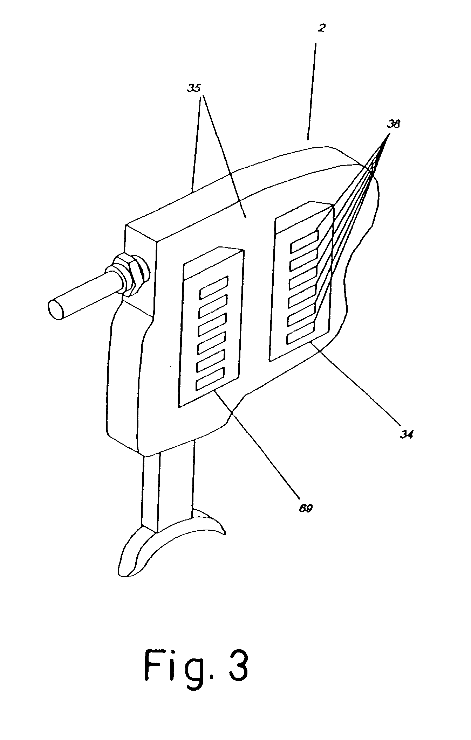

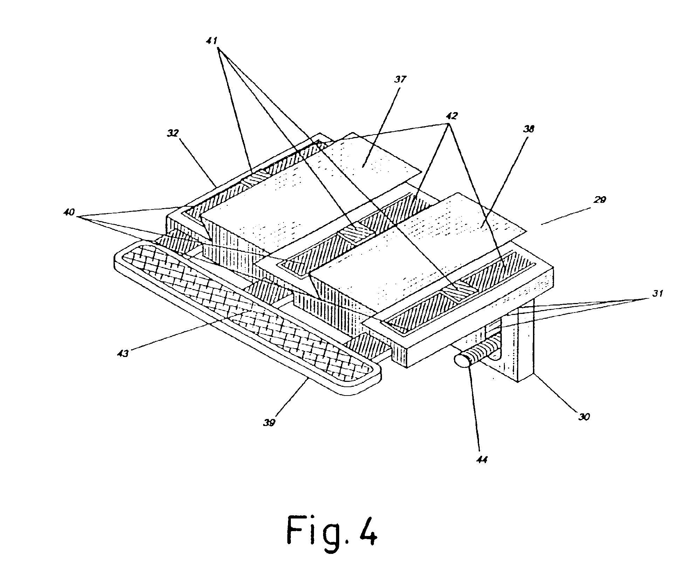

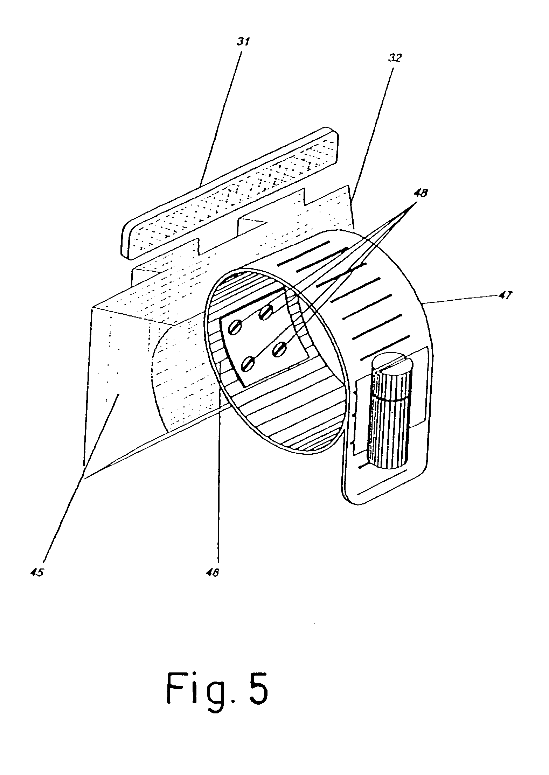

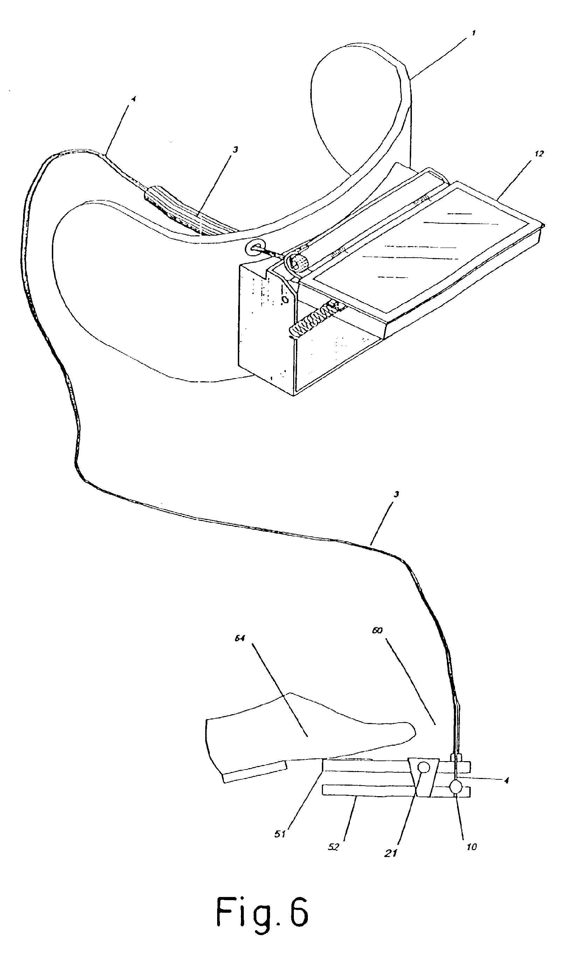

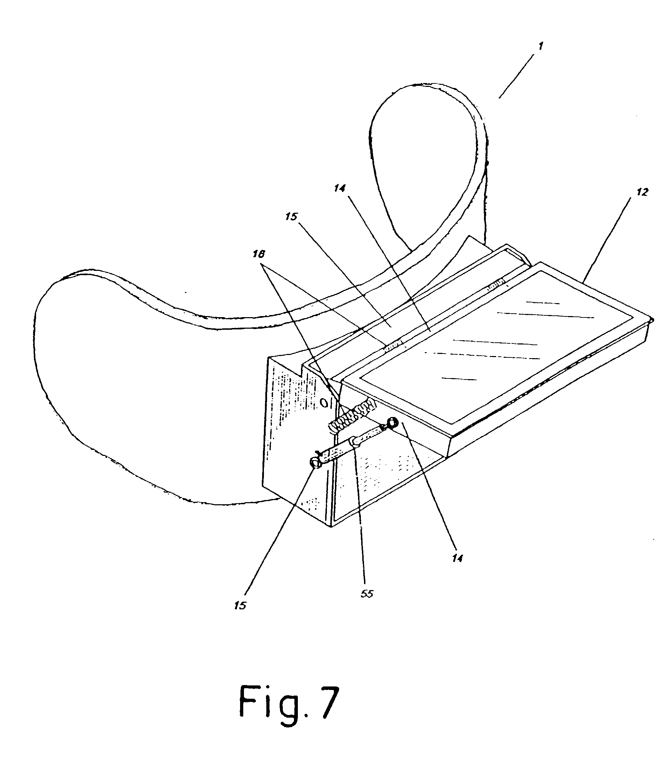

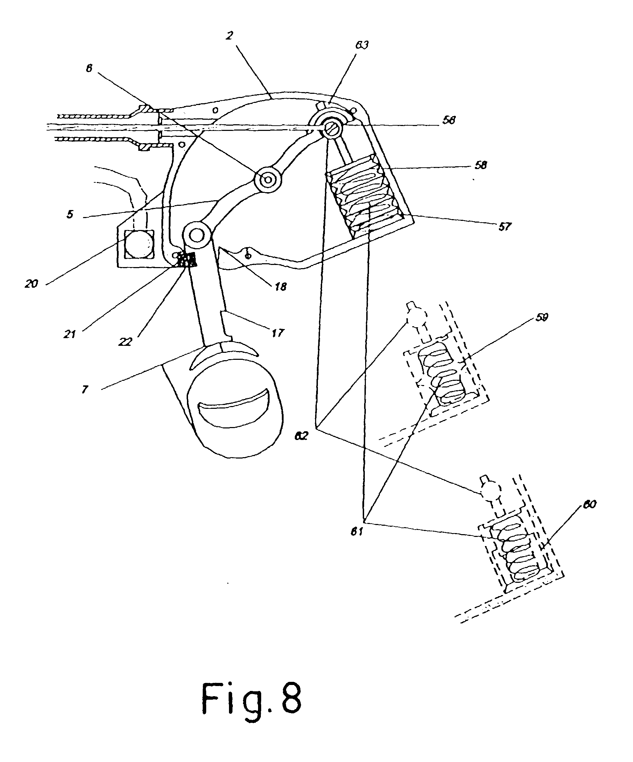

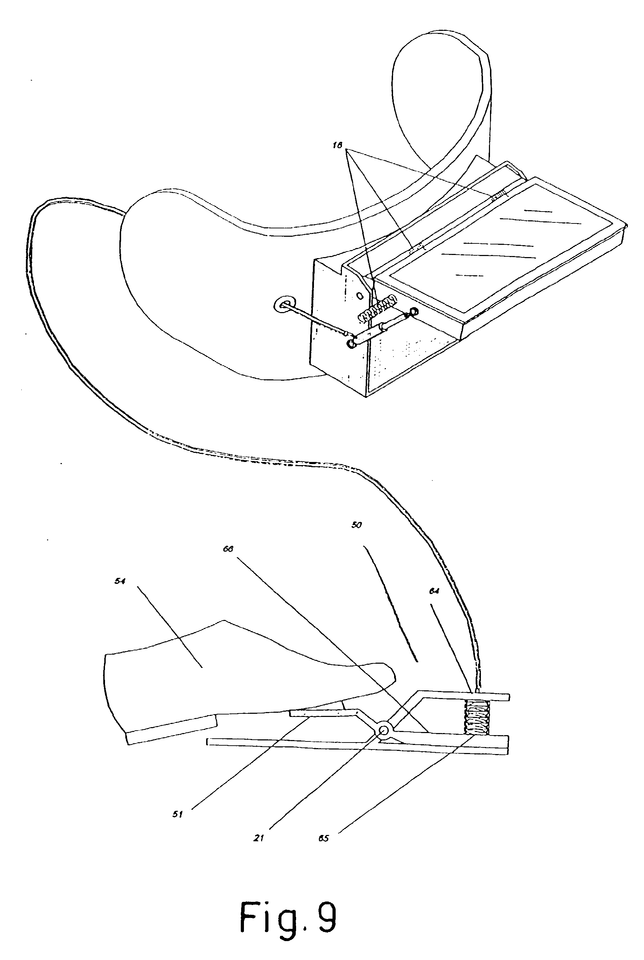

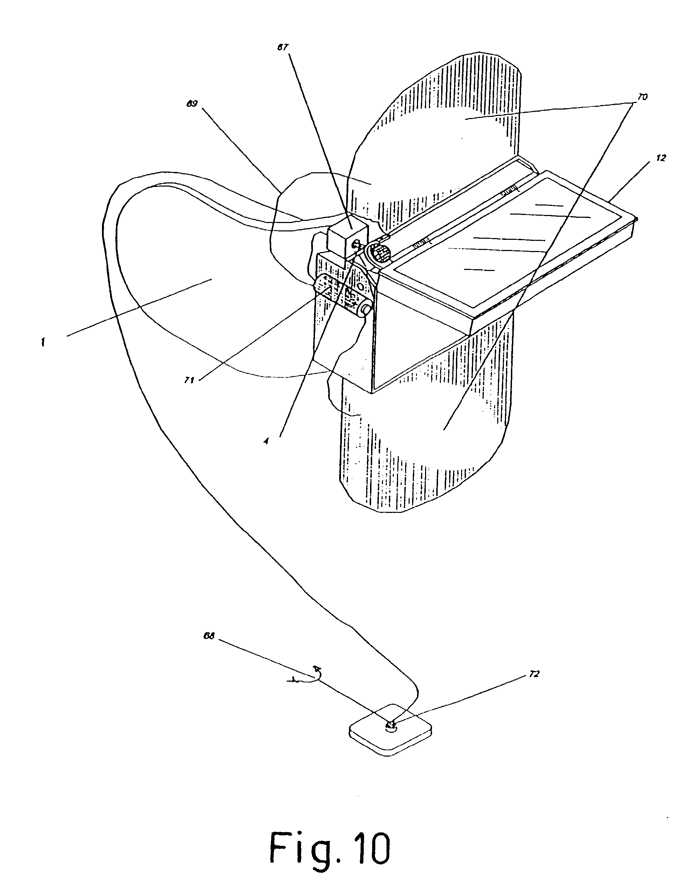

Figure # 10 shows a welding visual protector to weld (1) equal to that in Fig. 1, but different because here it is activated either by electromagnet (67) which by contracting plug (4) by means of electric (68) or solar (69) power received through solar panels (79) located in the top and bottom of the filter-holder window, which accumulates solar energy in batteries (71). It lifts up the filter-holder window (12) by applying the same principle as that in Fig. # 1, where a cylindrical tab with a slot, when plus is contracted with head-press, the plug lifts up the window (12) and then by means of a button (72) with wireless battery, for solar energy, located in the electrode-holder pliers the energy of electromagnet (67) is off, of the filter-holder window, and at the same time triggers the welding equipment contractor, restarting energy flow to welding pliers or cutting handle, and shuts under the same principle as that in Fig. By means of springs located between the base and the window.

## Claims

1. Sight protecting device for the application of welding, whose filter-carrying window (carrying dark glass) includes means to be lifted or opened by mechanical means and/or pneumatic means and/or electric means and/or solar means, activated manually or with the feet through devices with wire rope and/or bellows and/or diaphragm and/or double acting piston and/or electromagnet, which are fixed and graduated to a base which is fastened to the electrode carrying clip, the means to close the window is through contracting springs, the devices have security sensing devices, which disconnect the flow of the electrode-carrying clips while the window is not closed.

2. Sight protecting device for the application of welding whose filter carrying window includes means to be lifted or opened according to the first claim characterized because the window where the welding filter or sight protecting glass has a flange at the left or right upper part, which makes that the window is lifted supported on the hinge or pivoting point.

3. Sight protecting device for the application of welding whose filter carrying window includes means to be lifted or opened according to the first claim characterized because the sight protecting window which pivots on its hinge is lifted because of the expansion of a double acting piston fixed at one of the sides of the window at the base or frame.

4. Sight protecting device for the application of welding whose filter carrying window includes means to be lifted or opened according to the first, second and third claims where the window has contracting springs situated between the window and its base or frame, closing the window once the different devices are de-activated.

5. Sight protecting device for the application of welding whose filter carrying window includes means to be lifted or opened according to the first, second and fourth claims where the tops of the wire rope are given by sphere with screws which fasten the wire ropes or the electromagnet piston that goes through it, called wire rope pressing heads or piston pressing heads.

6. Sight protecting device for the application of welding whose filter carrying window includes means to be lifted or opened according to the first, second, fourth and fifth claims where the flange of the filter carrying window has a cylindric shape which allows the wire rope or piston pressing head to be situated in any position without further friction.

7. Sight protecting device for the application of welding whose filter carrying window includes means to be lifted or opened according to the first, second, fourth, fifth and sixth claims where the flange of the filter carrying window has a vertical passing notch which allows the window to be lifted and lowered without changing the directional axis of the electromagnet wire rope or piston.

8. Sight protecting device for the application of welding whose filter carrying window includes means to be lifted or opened according to the first, second, fourth, fifth, sixth and seventh claims characterized because the window is lifted by means of the contraction of the wire rope pressing head caused by a V shaped lever where it pivots in the centre and in one of the ends there is a piston which is pushed with the finger and makes the wire rope pressing head which goes in the hole of the other end of the V with the case to separate, contracting the window flange at the other end of the case through the wire rope pressing head.

9. Sight protecting device for the application of welding whose filter carrying window includes means to be lifted or opened according to the first, second, fourth, fifth, sixth and seventh claims where the filter carrying window is opened through a pedal device shaped as a lever in line which pivots at the centre and which, when pressed with the foot at one end, allows the other end to increase the space with its base, contracting the wire rope pressing head of the protector through the collecting action of the wire rope inside its case.

10. Sight protecting device for the application of welding whose filter carrying window includes means to be lifted or opened according to the first, third and fourth claims characterized because the sight protecting device window opening is established by air or oil pressure through a bellow (air) or a diaphragm (air or oil) or a piston (oil) which have equal cylindric shapes and are communicated with the double acting piston of the window through a hose.

11. Sight protecting device for the application of welding whose filter carrying window includes means to be lifted or opened according to the first, third, fourth and tenth claims which shows a device where the different pressure generating units are mounted, which has a horizontal lever which pivots in the centre and at one of its ends a pivot hangs which when introduced inside the device with the finger contracts the opposite end with the base, which is where the pressure units are situated.

12. Sight protecting device for the application of welding whose filter carrying window includes means to be lifted or opened according to the first, third, and tenth claims which has a pressure unit carrying device which is operated with the foot, pressing one of the lever ends which pivots in the centre on its base contracting the pressure units situated between the base and the other end of the lever.

13. Sight protecting device for the application of welding whose filter carrying window includes means to be lifted or opened according to the first, second, fourth, fifth sixth and seventh claims characterized because the window is lifted by means of an electromagnet situated behind and at the height of the window flange when its piston is contracted. The latter has a piston pressing head at one end supported on the flange, the energy flow is delivered by the energy of the equipment through cables or by means of solar panels situated at the upper and lower part of the window and which charge a battery which feeds the electromagnet.

14. Sight protecting device for the application of welding whose filter carrying window includes means to be lifted or opened according to the first, eighth and eleventh claims characterized because the manual mechanical means and the pressure generating manual unit carrying device has a hollow at the rear part of its piston boosting device, which been introduced into the piston by means of slight forward movement, fits in a disk of the device, allowing the piston to stay inside it and the window open and with another movement towards the rear it is disconnected.

15. Sight protecting device for the application of welding whose filter carrying window includes means to be lifted or opened according to the firs, eighth and eleventh claims characterized because the manual throttling devices both the mechanical one and the pressure generating carrying unit device have the same connecting system at the base which is fixed to the electrode carrying clip or the cutting handle, this base has two parallel trapezoidal bars which fits in with the device, creating the shape called dovetail. This base has three bars situated at the sides of the trapezoids, joined at an end to the base with a pin and at the other side to a button which, when introduced hides the pins, allowing to graduate and disconnect the devices from the base.

16. Sight protecting device for the application of welding whose filter carrying window includes means to be lifted or opened according to the first, eighth and eleventh claims where the different devices have two parallel trapezoidal bars at each side with rectangular perforations, six in each bar for its graduation with its base.

17. Sight protecting device for the application of welding whose filter carrying window includes means to be lifted or opened according to the previous claims from one to sixteen characterized because they can be built up of thermostable, thermobuildable or fibreglass plastic materials, which moreover has a metal covering.

18. Sight protecting device for the application of welding whose filter carrying window includes means to be lifted or opened according to the first, eighth and eleventh claims characterized because in the devices there is a wireless sensing device which signals to a contactor of the equipment to cut off the energy when the sensing device is not in front of its mirror situated in the piston and it connects the energy again when it is in front of its mirror, and this is when the window is closed and the piston is outside the device without being activated.
